# EUROPEAN PATENT APPLICATION

(11) **EP 1 162 267 A2**
(43) Date of publication of application: **12.12.2001**
(21) Application number: 01118243.3
(22) Date of filing: 31.03.1993
(51) Int. Cl.: C12N 15/40, C12N 15/82, C12N 15/83, C12N 7/01

(54) **Plant virus vector, plasmid, process for expression of foreign gene and process for obtaining foreign gene product**

(30) Priority: 31.03.1992 JP 10862892; 22.06.1992 JP 18874492; 08.12.1992 JP 35197092
(62) Divisional of application: 93906862.3
(71) Applicant: Kanebo Limited, Sumida-ku, Tokyo 131-0031 (JP)
(72) Inventor: Hamamoto, Hiroshi, Tsukuba-shi, Ibaraki (JP); Sugiyama, Yoshinori, Odawara-shi, Kanagawa (JP); Nakanishi, Noriyuki, Zama-shi, Kanagawa (JP); Nakagawa, Noriaki, Odawara-shi, Kanagawa (JP); Tsuchimoto, Suguru, Odawara-shi, Kanagawa (JP); Hashida, Eiji, Odawara-shi, Kanagawa (JP); Matsunaga, Yuji, Osaka-shi, Osaka (JP); Okada, Yoshimi, Matsudo-shi, Chiba (JP)
(74) Representative: Denison, Christopher Marcus

(57) **Abstract**

The present invention relates to a plant virus vector comprising a foreign gene linked downstream of a coat protein gene of tobacco mosaic virus via a nucleotide sequence which cause the readthrough, and a plasmid which is transcribed to provide the vector, as well as a process for expression of a foreign gene in a plant by inoculating the plant with the vector.

In addition, the present invention relates to a process for efficiently recovering a foreign gene product produced in a plant as virions.

SEQUENCE LISTING

(1) GENERAL INFORMATION:

(i) APPLICANT:
(A) NAME: Kanebo Limited
(B) STREET: 17-4 Sumida 5-chome, Sumida-ku
(C) CITY: Tokyo
(E) COUNTRY: Japan
(F) POSTAL CODE (ZIP): Tokyo 131

(ii) TITLE OF INVENTION: Plant virus vector, plasmid, process for
expression of foreign gene and process for obtaining
foreign gene product

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 93906862.3
(vi) PRIOR APPLICATION DATA:
(A) APPLICATION NUMBER: JP 4-108628
(B) FILING DATE: 31-MAR-1992

(vi) PRIOR APPLICATION DATA:
(A) APPLICATION NUMBER: JP 4-188744
(B) FILING DATE: 22-JUN-1992

(vi) PRIOR APPLICATION DATA:
(A) APPLICATION NUMBER: JP 4-351970
(B) FILING DATE: 08-DEC-1992

(2) INFORMATION FOR SEQ ID NO: 1:

(i) SEQUENCE CHARACTERISTICS:
(A) LENGTH: 66 base pairs
(B) TYPE: nucleic acid
(C) STRANDEDNESS: single
(D) TOPOLOGY: linear

2

(ii) MOLECULE TYPE: Other nucleic acid: Synthetic DNA

(xi) SEQUENCE DESCRIPTION: SEQ ID NO: 1:

GACCTCTGCA CCTGCATCTA GATTCTTCGT TGCTCCTTTT CCTGAAGTAT TCGGTAAGTA 60

AATGCA 66

(2) INFORMATION FOR SEQ ID NO: 2:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: Other nucleic acid: Synthetic DNA

(xi) SEQUENCE DESCRIPTION: SEQ ID NO: 2:

TTTACTTACC GAATACTTCA GGAAAAGGAG CAACGAAGAA TCTAGATGCA GGTGCAGAG 59

(2) INFORMATION FOR SEQ ID NO: 3:

(i) SEQUENCE CHARACTERISTICS:
(A) LENGTH: 75 base pairs
(B) TYPE: nucleic acid
(C) STRANDEDNESS: single
(D) TOPOLOGY: linear

(ii) MOLECULE TYPE: Other nucleic acid: Synthetic DNA

(xi) SEQUENCE DESCRIPTION: SEQ ID NO: 3:

GACCTCTGCA CCTGCATCTT AGCAATTAAG ATTCTTCGTT GCTCCTTTTC CTGAAGTATT 60

CGGTAAGTAA ATGCA 75

3

(2) INFORMATION FOR SEQ ID NO: 4:

(i) SEQUENCE CHARACTERISTICS:
(A) LENGTH: 68 base pairs
(B) TYPE: nucleic acid
(C) STRANDEDNESS: single
(D) TOPOLOGY: linear

(ii) MOLECULE TYPE: Other nucleic acid: Synthetic DNA

(xi) SEQUENCE DESCRIPTION: SEQ ID NO: 4:

TTTACTTACC GAATACTTCA GGAAAAGGAG CAACGAAGAA TCTTAATTGC TAAGATGCAG 60

GTGCAGAG 68

(2) INFORMATION FOR SEQ ID NO: 5:

(i) SEQUENCE CHARACTERISTICS:
(A) LENGTH: 75 base pairs
(B) TYPE: nucleic acid
(C) STRANDEDNESS: single
(D) TOPOLOGY: linear

(ii) MOLECULE TYPE: Other nucleic acid: Synthetic DNA

(xi) SEQUENCE DESCRIPTION: SEQ ID NO: 5:

GACCTCTGCA CCTGCATCAT AGCAATTAAG ATTCTTCGTT GCTCCTTTTC CTGAAGTATT 60

CGGTAAGTAA ATGCA 75

(2) INFORMATION FOR SEQ ID NO: 6:

(i) SEQUENCE CHARACTERISTICS:
(A) LENGTH: 68 base pairs
(B) TYPE: nucleic acid
(C) STRANDEDNESS: single
(D) TOPOLOGY: linear

(ii) MOLECULE TYPE: Other nucleic acid: Synthetic DNA

4

(xi) SEQUENCE DESCRIPTION: SEQ ID NO: 6:

TTTACTTACC GAATACTTCA GGAAAAGGAG CAACGAAGAA TCTTAATTGC TATGATGCAG 60

GTGCAGAG 68

(2) INFORMATION FOR SEQ ID NO: 7:

(i) SEQUENCE CHARACTERISTICS:
(A) LENGTH: 75 base pairs
(B) TYPE: nucleic acid
(C) STRANDEDNESS: single
(D) TOPOLOGY: linear

(ii) MOLECULE TYPE: Other nucleic acid: Synthetic DNA

(xi) SEQUENCE DESCRIPTION: SEQ ID NO: 7:

GACCTCTGCA CCTGCACAAT AGCAATTAAG ATTCTTCGTT GCTCCTTTTC CTGAAGTATT 60

CGGTAAGTAA ATGCA 75

(2) INFORMATION FOR SEQ ID NO: 8:

(i) SEQUENCE CHARACTERISTICS:
(A) LENGTH: 68 base pairs
(B) TYPE: nucleic acid
(C) STRANDEDNESS: single
(D) TOPOLOGY: linear

(ii) MOLECULE TYPE: Other nucleic acid: Synthetic DNA

(xi) SEQUENCE DESCRIPTION: SEQ ID NO: 8:

TTTACTTACC GAATACTTCA GGAAAAGGAG CAACGAAGAA TCTTAATTGC TATTGTGCAG 60

GTGCAGAG 68

5

(2) INFORMATION FOR SEQ ID NO: 9:

(i) SEQUENCE CHARACTERISTICS:
(A) LENGTH: 12 amino acids
(B) TYPE: amino acid
(D) TOPOLOGY: linear

(xi) SEQUENCE DESCRIPTION: SEQ ID NO: 9:

Phe Phe Val Ala Pro Phe Pro Glu Val Phe Gly Lys
1               5                   10

(2) INFORMATION FOR SEQ ID NO: 10:

(i) SEQUENCE CHARACTERISTICS:
(A) LENGTH: 7 amino acids
(B) TYPE: amino acid
(D) TOPOLOGY: linear

(xi) SEQUENCE DESCRIPTION: SEQ ID NO: 10:

Ala Val Pro Tyr Pro Gln Arg
1               5

(2) INFORMATION FOR SEQ ID NO: 11:

(i) SEQUENCE CHARACTERISTICS:
(A) LENGTH: 6 amino acids
(B) TYPE: amino acid
(D) TOPOLOGY: linear

(xi) SEQUENCE DESCRIPTION: SEQ ID NO: 11:

Thr Thr Met Pro Leu Trp
1               5

6

(2) INFORMATION FOR SEQ ID NO: 12:

(i) SEQUENCE CHARACTERISTICS:
(A) LENGTH: 5 amino acids
(B) TYPE: amino acid
(D) TOPOLOGY: linear

(xi) SEQUENCE DESCRIPTION: SEQ ID NO: 12:

Phe Phe Val Ala Pro
1               5

## Description

### FIELD OF THE ART

The present invention relates to introduction of a foreign gene to a plant using a plant virus vector, improvement of properties of plant and expression of a foreign gene through a whole plant, and process for simply and efficiently obtaining foreign gene product.

### BACKGROUND ART

It is known that a sort of plant viruses represented by tobamovirus etc., after infecting to a plant, proliferate in the plant and rapidly spread sytemically in plant while producing a protein called coat protein in a large amount.

So far, some gene engineering systems for these plant viruses, have been constructed, and some attempts have been carried out to introduce a foreign gene into a plant using said systems. For example, there are a process wherein a coat protein gene is replaced with a foreign gene (Takamatsu N., Ishikawa M., Meshi T. and Okada Y., EMBO J., 6, 307-311(1987)), and a process wherein a coat protein gene and a foreign gene are directly joined to as to produce a fused protein a: (Takamatsu N., Watanabe Y., Yanagi H., Meshi T. et. al., FEBS Lett., 269, 73-76(1990)) etc.

However, all of the plant viruses used in these known processes have a drawback in that they do not have an ability to spread systemically in plant (systemic infectivity), and it is impossible to introduce a useful property through a whole plant, and to produce a useful protein through a whole plant.

For a plant virus to exhibit the systemic infectivity, particle formation with wild type coat protein is essential (T. Saito et. al., Virology, 176, 329(1990)). As to existing plant virus vectors, it is considered that since coat protein is not produced (replacement type vector), or coat protein is in a form of a fused protein resulting in a big change of the properties (direct joining type vector), then particles cannot be formed and the systemic infectivity is not exhibited.

In addition, where existing plant virus vectors are used to express a foreign gene, it is very difficult to isolate and purify a foreign gene product from a plant into which foreign gene has been introduced.

The reason is as follow: to establish (1) simple apparatus and economy, (2) high recovery, (3) high purity and (4) good reproduction which are the goal of the isolation and purification, a combination of a plurality of operations such as differential precipitation, desalting, concentration, various chromatographies is essential; however a series of these operations usually take half a month or one month, and are often time- and labor-consuming. Even assuming these operations are simple and rapid, a plant includes proteins whose properties such as molecular weight, isoelectric point, affinity to a solvent, etc. are similar to those of the foreign gene product, and therefore it is difficult to prevent a loss of the foreign gene product during an isolation and purification process. Therefore, high recovery of the foreign gene product is not expected.

### DISCLOSURE OF THE INVENTION

Accordingly the purpose of the present inventions is to provide plant virus vectors having an ability to infect through a whole plant (systemic infectivity), plasmids which is transcribed to provide said plant virus vectors, and a process for expressing a foreign gene through a whole plant by inoculating said plant virus vector into a plant.

In addition, a process for simply and efficiently obtaining a foreign gene product produced in a plant is provided.

To accomplish the above-mentioned purposes, the present inventors enthusiastically carried out researches, and as a result, the use of a plant virus vector wherein a foreign gene is linked downstream of an coat protein gene of a plant virus via a nucleotide sequence which causes the readthrough (J.M. Skuzeski et. al., J. Mol. Biol. 218, 365-373(1991)), the use of plasmid which is transcribed to provide said plant virus vector, and the infection through a whole plant (systemic infectivity) by inoculating said plant virus vector into a plant so as to express the foreign gene through the whole plant become possible, and it was found that introduction of a useful property into the whole plant and the production of a useful protein in the whole plant are possible, and the present invention has been completed.

In addition the present inventors found that when a foreign gene product is recovered from a plant, the foreign gene product can be easily and efficiently recovered from plant as viral particles, and completed the present invention.

Next, prior to explaining the constituent features of the present invention, terms used in the specification are explained.

Plant virus vector: a replicable recombinant product obtainable by introducing a foreign gene into DNA or RNA sequence in a plant virus.

Readthrough: In a translation of an RNA to a protein, a phenomenon wherein the translation does not stop at a stop codon, and sometimes continues to a next stop codon. This phenomenon is caused by a nucleotide sequence near to the stop codon which is readthrough.

Plant: in addition to an individual plant (a plant consisting of leaves, stem and roots), plant cells, callus, protoplast, as well as adventous bud, adventious root and somatic embryo, derived from callus, are included, which are used as hosts for plant virus.

Wild coat protein: It means a protein essential for formation of virions. Although it is usually called simply coat protein, in the present invention, to distinguish it from a coat protein in a fused protein, it is described as wild coat protein.

Fused protein: It means a protein wherein a foreign gene product is joined to the C-terminal of a coat protein, or a protein wherein a part of a coat protein is replaced with a foreign gene product.

Virion: it means a viral genome covered with coat protein.

As plant viruses used in the present invention, there are mentioned a group belonging to DNA viruses, such as Caulimovirus, Geminivirus et. al., and a group belonging to RNA viruses, such as Tobamovirus, Bromovirus, Cucumovirus etc. Particular virus species include Cauliflower mosaic virus belonging to the Caulimovirus; Tomato golden mosaic virus belonging to the Geminivirus; Tobacco mosaic virus belonging to the Tobamovirus; Bromo mosaic virus belonging to the Bromovirus; Cucumber mosaic virus belonging to the Cucumovirus; and the like.

Foreign genes used in the present invention include genes for peptides having a pharmacological or physiological activity; genes for protein which provide stress resistance or pest resistance to a plant; and genes for proteins which change shape or flower color of a plant. More particularly, genes coding for enkephalins, calcitonins, corticotropins, human epidermal growth factor, and angiotensin converting enzyme inhibitor (ACEI) peptides having hypotensive action as shown in Table 1.

**Table 1**

| | |
|---|---|
| CEI₁₂: | Phe-Phe-Val-Ala-Pro-Phe-Pro-Glu-Val-Phe-Gly-Lys |
| CEI_{β7}: | Ala-Val-Pro-Tyr-Pro-Gln-Arg |
| CEI₆ : | Thr-Thr-Met-Pro-Leu-Trp |
| CEI₅ : | Phe-Phe-Val-Ala-Pro |

These genes may be derived from genomic DNA and cDNA of organisms, as well as plasmid DNAs, though they can be synthesized by a DNA synthesizer etc.

Nucleotide sequences used in the present invention, which cause the readthrough, may be any nucleotide sequences which cause the readthrough phenomenon, and include, for example, naturally occurring nucleotide sequences such as a nucleotide sequence present in a gene for protein called 130/180K of tobacco mosaic virus, and those nucleotide sequence wherein a part of said nucleotide sequence is replaced with other nucleotide sequence.

Particularly, there are mentioned UAGCAAUUA present in the 130/180K protein gene of tobacco mosaic virus, or a corresponding DNA type TAGCAATTA (wherein the underlined portions are a stop codon); UAACAAUUA (TAACAATTA) and UGACAAUUA (TGACAATTA) wherein said stop codon is replaced with other stop codon; and UAGCARYYA (TAGCARYYA) (wherein R represents A or G, Y represents C or U or T) wherein a portion other than the stop codon is replaced, though among them UAGCAAUUA (TAGCAATTA) is preferable because of high readthrough efficiency.

Coat protein genes of plant viruses used in the present invention include naturally occurring genes, genes wherein a part of naturally occurring gene is deleted, and genes wherein a part of naturally occurring gene is replaced with other nucleotide sequence, and particularly, coat protein genes whose nucleotide just before the readthrough-causing nucleotide sequence is A are preferable because of its high readthrough efficiency.

Concrete examples of replacement of nucleotide sequence are, in the case of tobacco mosaic virus, replacement of the U(T) of the 3'-terminus of coat protein gene with A, replacement of the UCU(TCT) of the 3'-terminus with CAA, and the like, and these replacements are preferable in that they remarkably increase readthrough efficiency.

Plasmids used in the present invention include, in the case where plant virus is RNA virus, for example, pLFW3 [Meshi T. et. al., Proc. Natl. Acad. Sci. USA, 83, 5043(1986)] having a PM promoter [Ahlquist. P. and Janda M., Mol. Cell. Biol., 4, 2876-2882(1984)] as a promotor for in vitro transcription; pTLW3 [Meshi T., Watanabe Y. and Okada Y., in: Genetic Engineering with Plant Viruses., Wilson, T.M.A. and Davis, J.W (Eds)., CRC, Florida, USA, p154(1992)] having T7 promotor [Rosa, M.D., Cell, 16, 815-825(1979)], though in the case where plant virus is DNA virus, plasmid not having promotor for in vitro transcription can be used.

Plant virus vectors, in the case where the virus is RNA virus, can be constructed, for example, as follow.

First, cDNA of a plant RNA virus is introduced into downstream of promotor of a plasmid having a promotor for in vitro transcription. Next, according to a conventional procedure used in a gene engineering, a desired foreign gene is introduced downstream of a coat protein gene of plant virus via a readthrough-causing nucleotide sequence.

In this case, it is preferable to insert a nucleotide sequence encoding amino acid(s) recognized by protease etc. between the readthrough-causing nucleotide sequence and the foreign gene so that a foreign gene-derived protein (or peptide) can be easily isolated. Amino acid recognized by protease etc. include, for example, arginine and lysine (cleaved with trypsin), phenylalanine and tyrosine (cleaved with chymotrypsin), as well as methionine (chemically cleaved with cyanogen bromide).

Next, RNA is prepared from the plasmid as constructed above by in vitro transcription, and is used as a plant virus vector. In addition, the present plant virus vector may be those prepared by forming particles with coat protein of a wild type virus (plant virus vector particles).

Where the virus is of DNA, DNA virus is directly introduced into a plasmid for gene engineering. A plant virus vector can be prepared by cutting out the DNA virus portion from the engineered plasmid, or a similar method.

The plant virus vector obtained as described above can be easily infected into a plant in a form of RNA or DNA, or preferably in a form of virion if it is RNA, for example by rubbing it on a leaf with carborundum, or by spraying it as a mixture with carborundum, or the like.

In this case, the plants may be any plant to which the present plant virus vector can infect, and for example, where the virus used as a vector is Tobacco mosaic virus, plants belonging to the family Solanacease, such as tobacco, tomato, red pepper etc. may be used; where the virus is Brom mosaic virus, plants belonging to the family Graminales such as barley, wheat etc. may be used; where the virus is cauliflower mosaic virus, plants or cultured cells belonging to the family Cruciferal such as cauliflower, turnip etc. may be used.

Infected plant simultaneously produces both the wild type coat protein and a fused protein through the whole plant. Namely, a foreign gene product is produced as a fused protein.

In addition, it can be found by isolating, plant various from infected plant and purifying and unolysing the plant virions that both the wild type coat protein and the fused protein are usedl4 for formation of various.

This fact means that the fused protein is also used for formation of virions in the copresence of the wild be obtained as a fused protein by recovering the plant virions from the plant. The fused protein thus obtained may be cleaved by an appropriate means to obtain a desired foreign gene product.

The recovery of virions does not require expensive reagents, is not time-consuming, and can be finished in approximately one day only by centrifugation. In addition, the reproductivity of centrifugation is high, and purity of virions obtained is high.

Since the present plant virus vector has a nucleotide sequence which causes the readthrough, it simultaneously produces both the wild type coat protein and a fused protein (a fused protein comprising a desired protein or peptide derived from a foreign gene and the coat protein). Therefore, virions are normally formed resulting in systemic infection and expression of the foreign gene through a whole plant.

In addition, according to the present invention, a foreign gene product with high purity can be reproducibly, easily and cheaply isolated and purified.

### BRIEF EXPLANATION OF DRAWINGS

Fig. 1 shows plasmid pTLW3.

Fig. 2 shows a nucleotide sequence of a synthetic DNA used for construction of plant virus vectors in Examples and Comparative Examples (A and A' were used in Comparative Example 1; B and B' were used in Example 1; C and C' were used in Example 2; and D and D' were used in Example 3).

Fig. 3 shows, in plasmid pTLW3, the positions of DNA fragments used for construction of plasmids in Examples and Comparative Examples.

Fig. 4(a) shows plasmid constructed in Comparative Example 1 and (b) shows plasmid constructed in Example 1.

Fig. 5 shows the presence or absence of virus vector in an infected leaf and non-infected leaf, in experiments for infection to an individual plant, carried out in Comparative Example 1 and Examples 1 to 3.

Fig. 6 shows the presence or absence of ACEI in an infected leaf and non-infected leaf, in experiments for infection to an individual plant, carried out in Comparative Example 1 and Examples 1 to 3.

Fig. 7 shows an ability of the present readthrough type vectors (Examples 1 to 3) to produce a fused protein.

Fig. 8 shows the presence or absence of ACEI in a tomato fruit, in an experiment for infection to minitomato plant, carried out in Example 4.

Fig. 9 shows the purity of virions obtained in an experiment for recovering virions carried out in

### Example 5.

Fig. 10 represents the presence or absence of a fused protein and an ACEI peptide in virions obtained in an experiment for recovering virions, carried out in Example 5. (a) shows a result of antibody staining with anti-TMV rabbit serum, and (b) shows a result of antibody staining with anti-ACEI rabbit serum.

### BEST MODE FOR CARRYING OUT THE INVENTION

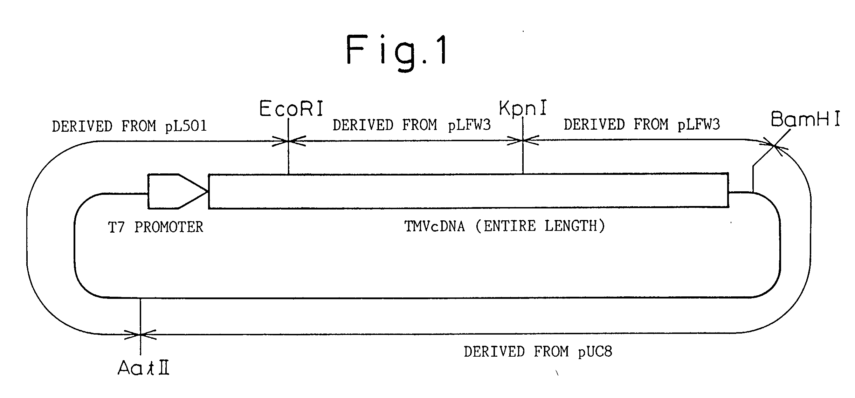

The present invention is further explained in detail in the following Examples. Note that plasmid pTLW3 used in Examples is shown in Fig. 1.

### Examples 1 to 3 and Comparative Example 1

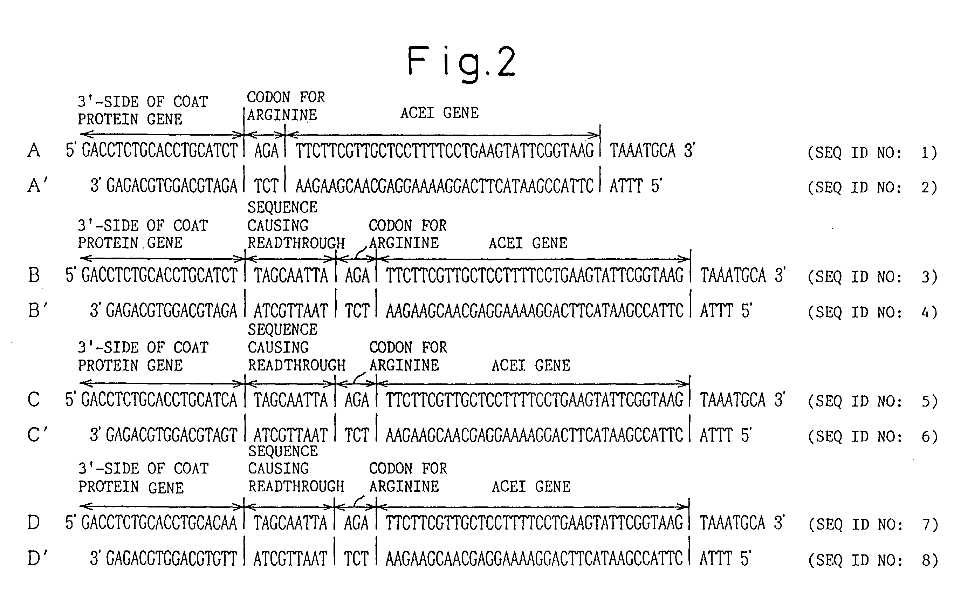

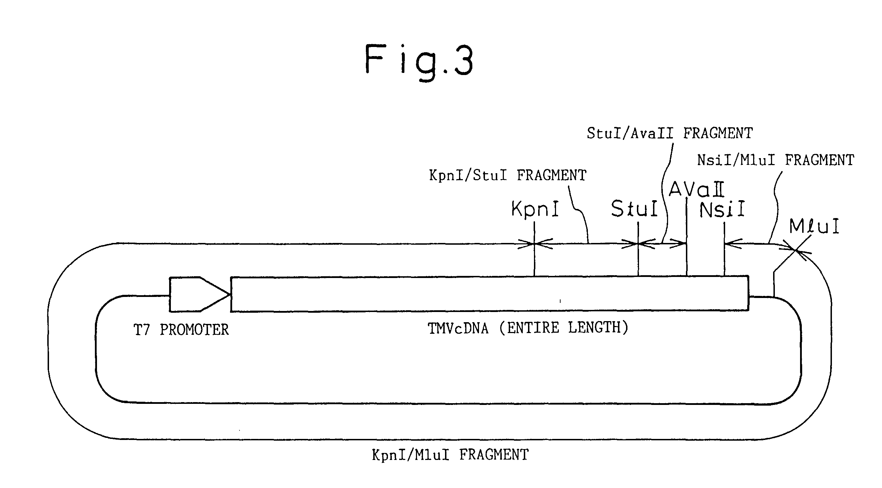

1) Construction of Template Plasmid Incorporating ACEI Gene Sequence
(1) Construction of Synthetic DNAs Comprising ACEI Gene Sequence
Eight synthetic DNAs shown in Fig. 2 were prepared in 0.2 µmol scale using a 380A Type DNA synthesizer of Applied Biosystems, Inc. (ABI Inc.). A and A' were used for conventional direct joined type vector (Comparative Example 1), B and B' were used for a readthrough type vector (Example 1), C and C' were used for a readthrough type vector wherein one nucleotide at the 3-terminus of a coat protein gene has been replaced (Example 2), and D and D' were used for a readthrough type vector where three nucleotides at the 3'-terminus of a coat protein gene were replaced (Example 3). All the vectors contain a sequence coding for an arginine at the 5'-side of an ACEI gene sequence so that an ACEI peptide can be isolated from an expressed fused protein by trypsin digestion. The synthesized DNAs were purified an OPC cartridge of ABI Inc.
(2) Phosphorylation and Annealing of the Synthetic DNAs
The DNAs synthesized in the above-mentioned (1) were adjusted to a concentration of 0.1 µg/µl, and phosphorylated with 0.2 mM ATP, and purified by treatment with phenol and washing with ethyl ether. The purified DNA was adjusted to a concentration of 0.02 µg/µl, and A and A', B and B', C and C', as well as D and D' were mixed, and each mixture was incubated at 65°C for 5 minutes. After the incubation, the mixture was allowed to gradually cooled to a room temperature for annealing.
(3) Preparation of Fragments for Construction of Plasmids
The plasmid pTLW3 shown in Fig. 1, which was constructed according to a published literature [Meshi T. et. al., in: Genetic Engineering with plant Viruses, Wilson, T.M.A. and Davis, J.W. (Eds.)., CRC, Florida, USA., p154(1992)], was digested with a combination of restriction enzymes shown in Table 2 to prepare fragments having different sizes. Note, the relationship between the fragments in pTLW3 is shown in Fig. 3.

**Table 2**

| | |
|---|---|
| KpnI/MluI Fragment | 7.1 kbp |
| KpnI/StuI Fragment | 1.2 kbp |
| StuI/AvaII Fragment | 0.56 kbp |
| NsiI/MluI Fragment | 0.2 kbp |

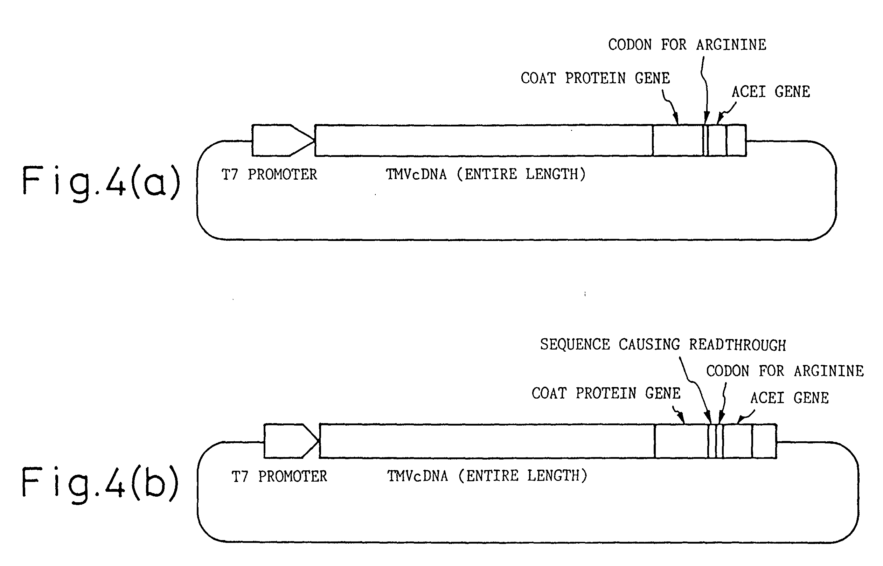

After separation by agarose electrophoresis, each fragment was prepared using a GENE CLEAN kit (BIO 101) according to the instructions attached to the kit.
Among the above-mentioned fragments, the KpnI-MluI 7.1 kbp fragment was dephosphorylated at its end by alkaline phosphatase treatment, and used in the following reaction.
(4) Construction and Preparation of Plasmid Each of 4 solutions of the annealed synthetic DNAs prepared in the above-mentioned (2) was reacted with a solution of 4 DNA fragments derived from pTLW3 prepared in the above-mentioned (3) respectively at 15°C for 15 hours in the following composition so as to construct 4 plasmids. Among the plasmids thus obtained, two plasmids are shown is Fig. 4 (a: Comparative Example 1; b: Example 1).

**Table 3**

| | |
|---|---|
| Solution of annealed synthetic DNA (0.02 µg/µl) | 1.0 µl |
| KpnI/MluI Fragment (0.001 µg/µl) | 9.0 µl |
| KpnI/StuI Fragment (0.01 µg/µl) | 1.5 µl |
| StuI/AvaII Fragment (0.015 µg/µl) | 0.5 µl |
| NsiI/M1uI Fragment (0.005 µg/µl) | 0.5 µl |
| ×10 binding buffer | 1.5 µl |
| T4 Ligase [Takara Shuzo 350 Units/µl] | 1.0 µl |
| Total | 15.0 µl |

Note, the composition of the ×10 binding buffer is shown in Table 4.

**Table 4**

| Composition of ×10 binding buffer | |
|---|---|
| Tris-HC1 (pH=7.6) | 660 mM |
| MgCl₂ | 66 mM |
| DTT | 100 mM |
| ATP | 1 mM |

The reaction mixture as such was used to transform competent cells of E. coli HB101 (Takara Shuzo), and colonies resistant to carbenicilin were selected. A desired plasmid was extracted and purified from E. coli cells of the selected calories.
2) Construction of Plant Virus Vectors by in Vitro Transcription
Four plasmids constructed in the above-mentioned 1) (a plasmid having a sequence of a conventional vector, and plasmids having sequence of vector of the present invention) were digested with restriction enzyme, and purified by phenol treatment and ethanol precipitation, and used as template DNAs. In vitro transcription reaction was carried out using said template DNA in the reaction system shown in Table 5, at 37°C for 2 hours to construct four plant virus vectors (RNA). The plant virus vectors (RNA) thus constructed were purified by phenol treatment and ethanol precipitation.

**Table 5**

| | |
|---|---|
| 1M Tris-HCl (pH=8.0) | 4 µl |
| 1M MgCl₂ | 2 µl |
| 1M DTT | 1 µl |
| 10 mg/ml BSA | 1 µl |
| 100 mM ATP | 1 µl |
| 100 mM UTP | 1 µl |
| 100 mM CTP | 1 µl |
| 10 mM GTP | 2 µl |
| 10 mM CAP | 20 µl |
| Template DNA (0.5 mg/ml) | 10 µl |
| T7 Polymerase [Takara Shuzo 50 Units/µl] | 1 µl |
| RNase Inhibitor [Takara Shuzo 50 Units/µl] | 1 µl |
| | |
| H₂O | 55 µl |
| Total | 100 µl |

The plant virus vector (RNA) prepared as above and wild type coat protein of tobacco mosaic virus purified according to a known procedure [Frankel-Contrat H., Virology, 4, 1 to 4 (1957)] were reacted in the composition shown in Table 6, at 20°C for 15 hours artificially to form virions in vitro. The reaction mixture as such was used as plant virus vector particle solution.

**Table 6**

| | |
|---|---|
| Plant virus vector (RNA) (0.3 µg/µl) | 8 µl |
| Wild type coat protein (10 µg/µl) | 10 µl |
| 0.2 M Phosphate buffer (pH=7.0) | 18 µl |

3) Introduction of Foreign Gene into Individual Plant by Inoculation of Plant Virus vector Particles
The solution of the plant virus vector particles prepared in the above-mentioned 2) was diluted 100 folds with 10 mM phosphate buffer (pH 7.0), and 100 µl of the diluted solution was rubbed on each leaf of Nicotiana tabacum cv. Samsun of 6 weeks old from seeding using carborundum (Nakarai Tesk, Carborundum, 600 mesh).
4) Confirmation of Replication of Plant Virus vector in Individual Plant and Movement to Upper Laves
(1) Preparation of Sample
After 15 days from the infection of plant virus vector particles, 10 mg of sections of an infected leaf and of non-infected leaf from the same individual plant were cut off. To the leaf section was added 50 µl of an SDS sampling buffer having the composition shown in Table 7, and after homogenizing, the homogenate was treated at 100°C for 3 minutes. After cooling, the homogenate was centrifugal at 12,000 rpm for 5 minutes, and the supernatant was used as an SDS-protein sample.

**Table 7**

| Composition of SDS sampling buffer | |
|---|---|
| Tris-HCl (pH=6.8) | 120 mM |
| SDS | 4% |
| 2-Mercaptoethanol | 10% |
| Glycerol | 20% |
| BPB | 0.002% |

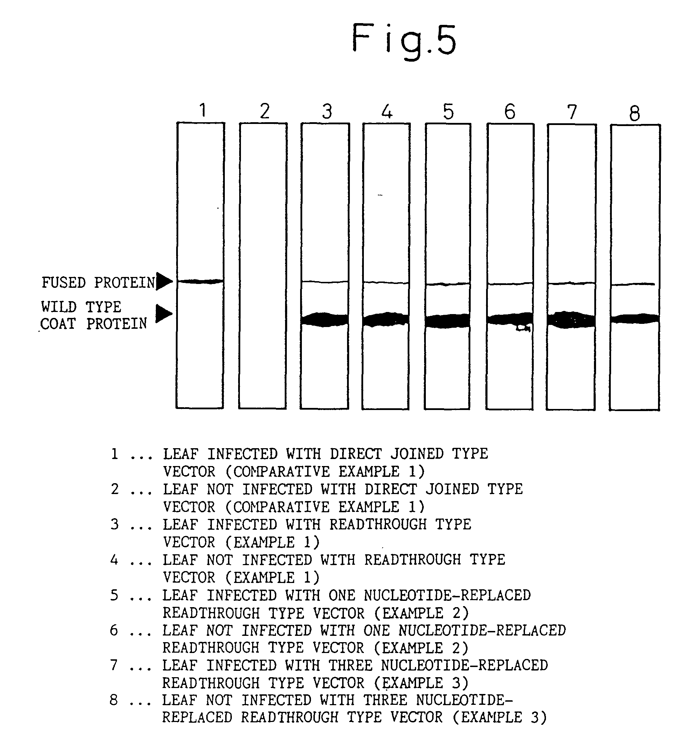

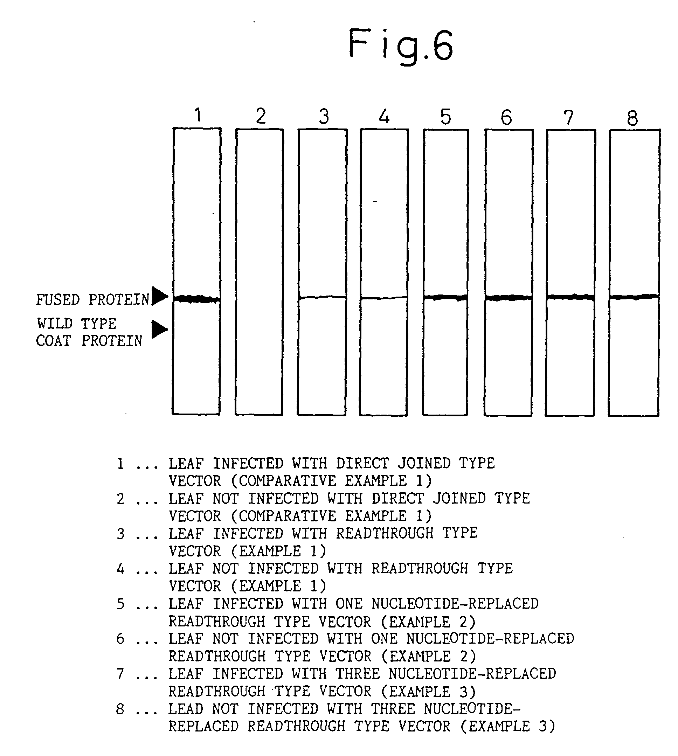

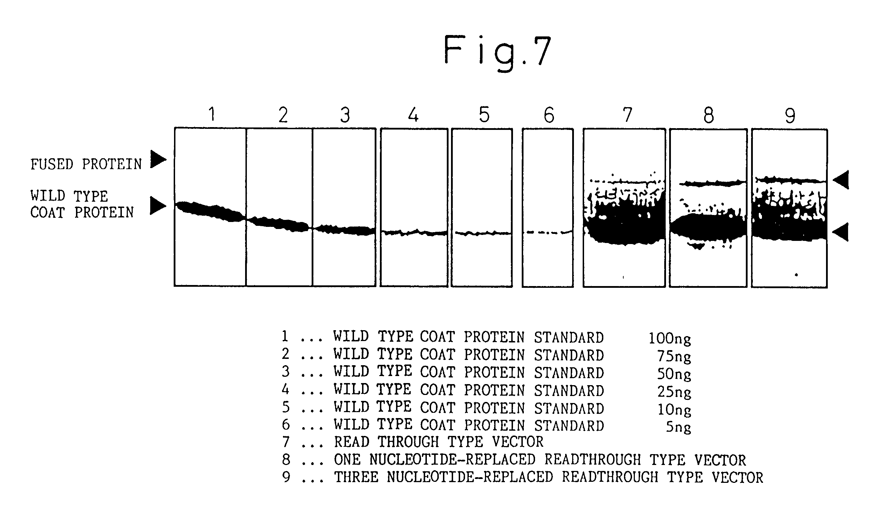

(2) Detection of Plant Virus Vector in Sample
The presence or absence of plant virus vector was determined by the presence or absence of wild type coat protein and a fused protein. One µl of the SDS-protein sample prepared in the above-mentioned (1) was subjected to SDS-PAGE (12.5% polyacrylamide gel) to separate proteins. The separated proteins were transferred from the gel to a PVDF membrane by electric blotting. Next, an anti-tobacco mosaic virus rabbit serum was diluted 64,000 folds with TBS buffer [20 mM Tris-HCl (pH 7.6) and 137 mM NaCl] to obtain a primary antibody staining solution, and antibody staining was carried out using a rabbit antibody blotting detection kit (Amersham) according to the instructions attached to the kit. A result is shown in Fig. 5.
Although the conventional type plant virus vector producing a fused protein alone (Comparative Example 1) was detected in an infected leaf, the movement to non-infected leaves was not observed (Fig. 5, lanes 1 and 2).
On the other hand, the present plant virus vector into which a readthrough sequence had been introduced so that both the wild type coat protein and a fused protein were simultaneously produced (Examples 1 to 3) was detected in both the infected leaf and non-infected leaves, which confirmed that the vector was spread from a leaf to another leaf (i.e., it had systemic infectivity) (Fig. 5, lanes 3 to 8).
5) Confirmation of Production of ACEI in Plant
According to the same procedures as described in the above-mentioned 4), the sample of 4) was subjected to electrophoresis and transfer to the membrane, and using an anti-ACEI rabbit serum diluted 128,000 folds with TBS buffer as a primary antibody staining solution, antibody staining was carried out. A result is shown in Fig. 6.
It was confirmed that the conventional type plant virus vector produced ACEI in a form of a fused protein only in an infected leaf, and the present plant virus vectors produce ACEI in a form of a fused protein in both an infected leaf and non-infected leaf.
6) Comparison of Fused Protein Productivity of Plant Virus vectors
The same procedure as described in (2) was carried out to compare the fused protein productivity of plant virus vectors of Examples 1 to 3.
In this case, samples to be subjected to SDS-PAGE were extracted from infected leaves, and an amount of the sample was adjusted to correspond to 0.2 mg of a live leaf. In addition, as a standard, 100, 75, 50, 25, 10, and 5 ng of wild type coat protein of tobacco mosaic virus was simultaneously subjected to the SDS-PAGE. Similarly to 4), (2) above, the detection was carried out by antibody staining with anti-tobacco mosaic virus rabbit serum. A result is shown in Fig. 7.
When an amount of a fused protein produced by each vector was calculated by comparing the color densities of bands in Fig. 7, the vector of Example 1 produced 0.025 mg fused protein per 1g tobacco leaf, and the vectors of Examples 2 and 3 produced 0.25 mg fused protein per 1g tobacco leaf. From this result, it was found that replacement of U with A or UCU with CAA at the 3'-terminus of coat protein gene enhances an efficiency of the readthrough, and increases a fused protein productivity by 10 times.

### Example 4

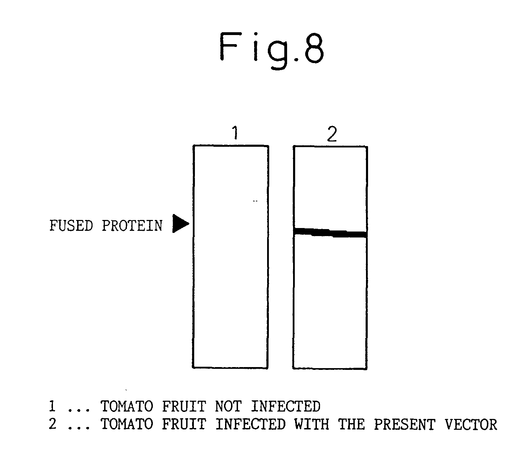

A solution of the present plant virus vector particles having ACEI gene sequence constructed in Example 1 was infected to a leaf of minitomato plant [Sugarlamp purchased from SAKATA NO TANE] about 6 months old from the seeding. The infection was carried out as in Example 1. After 14 days, tomato fruit was harvested, and ACEI in a protein sample extracted from the tomato fruit was detected using an anti-ACEI antibody according to the same procedure as described in Example 1. A result is shown in Fig. 8.
As can be seen from Fig. 8, it was confirmed that ACEI was produced in a tomato fruit.
It is expected that the tomato fruit produced contains a large amount of ACEI which exhibits hypotensive action by oral uptake.

### Example 5

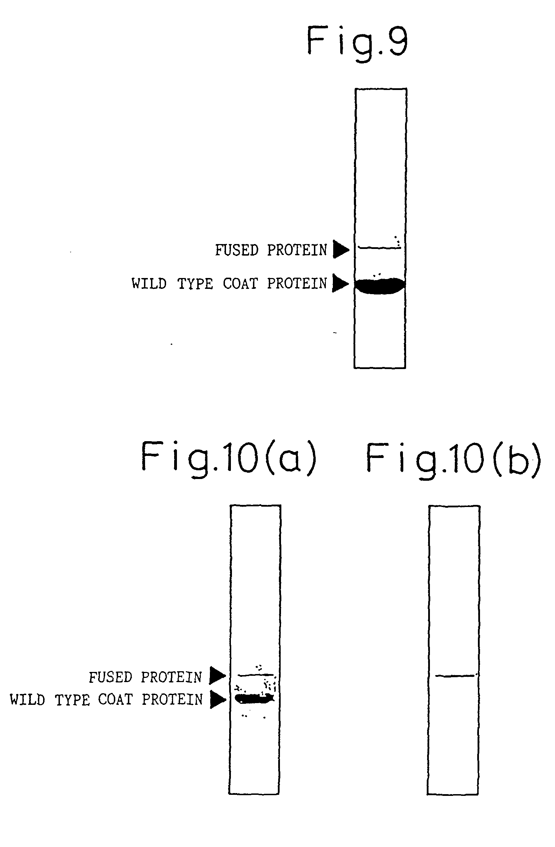

1) Introduction of Foreign Gene into Plant by Inoculation of Plant Virus vector
   A solution of the present plant virus vector particles having ACEI gene sequence constructed in Example 2 was infected to a leaf of Nicotiana tabacum cv. Samsun, 6 weeks old from the seeding. The infection was carried out as in Examples 1 or 2.
2) Recovery of Plant Virions from Plant Incorporating a Foreign Gene
   After 1 to 2 months from the infection, virions were purified from 10g of leaves of the infected plant. More specifically, the following procedures were carried out.
   Non-infected leaves of the infected tobacco plant were frozen and disrupted with a mixer. Next, to the disrupted non-infected leaves was added the same weight of 0.1M phosphate buffer (pH 7.0) containing 0.1% thioglycolic acid, and after homogenizing the mixture, the homogenate was centrifuged at a low temperature and a low speed (8000×g, 10 minutes). The precipitate was discarded, and to the supernatant, were added 2M sodium chloride solution in an amount of 0.06 volume relative to the volume of the supernatant, and 20% polyethylene glycol in an amount of 0.2 volume relative to the volume of the supernatant, and the mixture was allowed to stand on ice for one hour.
   Next, the mixture was centrifuged at low speed to recover the precipitate. The precipitate was thoroughly dispersed in distilled water, and a differential centrifugation comprising low speed centrifugation and high speed centrifugation (100,000×g, 50 minutes) was twice repeated. The precipitate from the final centrifugation was suspended in distilled water to obtain virions.
   Concentration of the virions was calculated as 10 mg/ml from absorbance at the wavelength 280 nm. The isolation and purification procedure described above took about one day.
3) Evaluation of purity of the virious
   To 5 µl of the virions was added 5 µl of an SDS sampling buffer having the composition shown in Table 7, and after treating at 100°C for 5 minutes, the mixture was used as an SDS-protein sample. Two µl of the SDS-protein sample was subjected to SDS-PAGE (12.5% polyacrylamide gel), and the gel was stained with Coomassie Brilliant Blue, decolared with 7.5% acetic acid/5% methanol, and dried. A result is shown in Fig. 9.
   As can be seen from Fig. 9, only the wild type coat protein and the fused protein were detected, and other proteins derived from the plant were almost completely eliminated.
4) Detection of fused protein in virions
   An SDS-protein sample was prepared according to the same procedure as described above 3), and diluted 10 folds, and 2 µl of the diluted sample was subjected to SDS-PAGE (12.5% polyacrylamide gel) to separate proteins. The separated proteins were transferred from the gel to a PVDF membrane by electro blotting.
   Next, antibody staining was carried out using an anti-TMV rabbit serum or anti-ACEI rabbit serum as a primary staining solution, and using a rabbit antibody blotting detection kit (Promega) according to the instructions attached to the kit. A result is shown in Figs. 10(a) and (b).
   A ratio of fused protein and wild type coat protein incorperated in the virions was about 1:20, and the ratio substantially conformed to that in proteins isolated and purified from the plant. This fact shows that the fused protein was efficiently incorporated into the virions.
   In addition, since the virions derived from non-infected leaf, contained a fused protein, it was found that by using a readthrough vector, virions containing a fused protein can be recovered from the whole plant.

### [Industrial Applicability]

As can be seen from the above, since the present plant virus vectors have a big advantage of systemic infectivity, then introduction of a desired property into a plant and the production of a foreign gene product in a whole plant are possible.

In addition, according to the present process, a foreign gene product with high purity can be reproducibly, cheaply and easily isolated and purified.

SEQUENCE LISTINGS
SEQ ID NO: 1
Length of sequence: 66
Type of sequence: Nucleic acid
Strandness: Single
Type of molecule: Synthetic DNA
Sequence
GACCTCTGCA CCTGCATCTA GATTCTTCGT TGCTCCTTTT CCTGAAGTAT 50
TCGGTAAGTA AATGCA 66
SEQ ID NO: 2
Length of sequence: 59
Type of sequence: Nucleic acid
Strandness: Single
Type of molecule: Synthetic DNA
Sequence
TTTACTTACC GAATACTTCA GGAAAAGGAG CAACGAAGAA TCTAGATGCA 50
GGTGCAGAG 59
SEQ ID NO: 3
Length of sequence: 75
Type of sequence: Nucleic acid
Strandness: Single
Type of molecule: Synthetic DNA
Sequence
GACCTCTGCA CCTGCATCTT AGCAATTAAG ATTCTTCGTT GCTCCTTTTC 50
CTGAAGTATT CGGTAAGTAA ATGCA 75
SEQ ID NO: 4
Length of sequence: 68
Type of sequence: Nucleic acid

Strandness: Single
Type of molecule: Synthetic DNA
Sequence
TTTACTTACC GAATACTTCA GGAAAAGGAG CAACGAAGAA TCTTAATTGC 50
TAAGATGCAG GTGCAGAG 68
SEQ ID NO: 5
Length of sequence: 75
Type of sequence: Nucleic acid
Strandness: Single
Type of molecule: Synthetic DNA
Sequence
GACCTCTGCA CCTGCATCAT AGCAATTAAG ATTCTTCGTT GCTCCTTTTC 50
CTGAAGTATT CGGTAAGTAA ATGCA 75
SEQ ID NO: 6
Length of sequence: 68
Type of sequence: Nucleic acid
Strandness: Single
Type of molecule: Synthetic DNA
Sequence
TTTACTTACC GAATACTTCA GGAAAAGGAG CAACGAAGAA TCTTAATTGC 50
TATGATGCAG GTGCAGAG 68
SEQ ID NO: 7
Length of sequence: 75
Type of sequence: Nucleic acid
Strandness: Single
Type of molecule: Synthetic DNA
Sequence
GACCTCTGCA CCTGCACAAT AGCAATTAAG ATTCTTCGTT GCTCCTTTTC 50

CTGAAGTATT CGGTAAGTAA ATGCA 75
SEQ ID NO: 8
Length of sequence: 68
Type of sequence: Nucleic acid
Strandness: Single
Type of molecule: Synthetic DNA
Sequence
TTTACTTACC GAATACTTCA GGAAAAGGAG CAACGAAGAA TCTTAATTGC 50
TATTGTGCAG GTGCAGAG 68

## Claims

1. A virion particle comprising a coat protein of a coat protein of a Tobamovirus and a fusion protein of the coat protein and a foreign protein.

2. A virion particle according to claim 1 wherein the coat protein gene is a coat protein a part of which is lacking or replaced.

3. A process for systemically expressing a fusion protein of a coat protein of a Tobamovirus and a foreign protein in a plant comprising the steps of:
(a) inoculating a plant with a plant virus vector, such that upon expression of the vector in a plant, the coat protein of a Tobamovirus and the fusion protein of the coat protein and the foreign protein are systemically produced in the plant; and
(b) expressing the fusion protein systemically in the plant.

4. A process according to claim 3 wherein the plant is tobacco or tomato.

5. A process for systemically expressing a fusion protein of a coat protein of a Tobamovirus and a foreign protein in a plant comprising the steps of:
(a) inoculating a plant with a plant virus vector, such that upon expression of the vector in a plant, the coat protein of a Tobamovirus and the fusion protein of the coat protein and the foreign protein are systemically produced in the plant;
(b) expressing the fusion protein systemically in the plant;
(c) isolating the foreign gene product from the virions.

6. A plant virus vector comprising a foreign gene linked to downstream of a coat protein gene via a nucleotide sequence which causes the readthrough.

7. A plant virus vector according to claim 6 wherein the coat protein gene is a coat protein gene a part of which is lacked or replaced.

8. A plant virus vector according to claim 6 or 7, wherein the plant virus is a DNA virus.

9. A plant virus vector according to claim 6 or 7, where the plant virus is an RNA virus.

10. A plant virus vector according to claim 8, wherein, the DNA virus is Caulimovirus or Geminivirus.

11. A plant virus vector according to claim 9, wherein the RNA virus is Tobamovirus, Bromovirus, Cucumovirus, or the like.

12. A plant virus vector according to claim 9, wherein the RNA virus is Tobamovirus.

13. A plant virus vector according to claim 6 or 7, wherein the nucleotide sequence which causes the read through is UAGCAAUUA or the DNA type thereof TAGCAATTA, UAACAAUUA or the DNA type thereof TAACAATTA, or UGACAAUUA or the DNA type thereof TGACAATTA, or UAGCARYYA or the DNA type thereof TAGCARYYA, wherein the underline indicates a stop codon, R is A or G, and Y is C or U or T.

14. A plasmid comprising a DNA sequence of a plant virus vector according to any one of claims 6 to 8, 10, and 13.

15. A plasmid comprising a DNA complementary to a plant virus vector according to any one of claims 6, 7, 9 and 11 to 13, and a promoter for transcribing said DNA.

16. A plasmid according to claim 15, wherein the promoter for transcribing the plasmid is PM promoter or T7 promoter.

17. A process for expressing a foreign DNA in a plant, **characterised by** inoculating a plant with a plant virus vector according to any one of claims 6 to 13, and expressing the foreign gene in the plant.

18. A process according to claim 17, wherein when the virus is Caulimovirus, the plant is turnip, cauliflower etc.

19. A process according to claim 17, wherein when the virus is Geminivirus, the plant is tomato, cassava, corn, etc.

20. A process according to claim 17, wherein when the virus is Tobamovirus, the plant is tobacco, tomato etc.

21. A process according to claim 17, wherein when the virus is Bromovirus, the plant is wheat, barley etc.

22. A process according to claim 17, wherein when the virus is Cucumovirus, the plant is cucumber, tomato etc.

23. A process for production of a foreign gene product, comprising the steps of:
(a) inoculating a plant with a plant virus vector according to any one of claims 6 to 13, and expressing the foreign gene in the plant virus vector;
(b) recovering virions from the plant; and
(c) isolating the foreign gene product from the virions.
